# EUROPÄISCHE PATENTSCHRIFT

(11) **EP 1 154 461 B1**
(45) Veröffentlichungstag und Bekanntmachung des Hinweises auf die Patenterteilung: **15.03.2006**
(21) Anmeldenummer: 01111543.3
(22) Anmeldetag: 11.05.2001
(51) Int. Cl.: H01J 61/42, H01J 61/44, H01J 61/46, A61N 5/06

(54) **Verwendung einer Edelgas-Niederdruck-Entladungslampe für kosmetische oder therapeutische Zwecke**
Cosmetic or therapeutic use of a noble gas low-pressure discharge lamp
Utilisation cosmétique ou thérapeutique d'une lampe à décharge basse pression à gaz rare

(30) Priorität: 13.05.2000 DE 10023504
(43) Veröffentlichungstag der Anmeldung: 14.11.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Jüstel, Thomas, Dr., 52064 Aachen (DE); Nikol, Hans, Dr., 52064 Aachen (DE); Jalink, Cornelis Jojakim, 52064 Aachen (DE)
(74) Vertreter: Volmer, Georg

(56) Entgegenhaltungen:
- EP-A- 1 067 166
- DE-A- 3 729 711
- DE-A- 19 737 920
- DE-U- 20 004 732
- US-A- 4 048 537
- US-A- 5 866 984

## Beschreibung

Die Erfindung betrifft eine Edelgas-Niederdruck-Entladungslampe, ein Verfahren zum Herstellen einer Edelgas-Niederdruck-Endadungslampe Lampe sowie verschiedene Verwendungen einer Gasentladungslampe insbesondere für kosmetische oder therapeutische Zwecke.

Edelgas-Niederdruck-Entladungslampen sind bekannt. Sie dienen z.B. zur Hintergrundbeleuchtung bei LCD-Displays. Daneben ist aus der WO 98/19327 eine Xenon-Lampe bekannt, die für bestimmte therapeutische Zwecke Licht mit eng begrenzten Spektrallinien abgibt. Für breite Einsatzzwecke, insbesondere als Bräunungslampe in Solarien eignet sich diese Lampe jedoch nicht.

Als Bräunungslampen sind bislang zum einen Quecksilber-Niederdruck-Gasentladungslampen bekannt, die mit einem oder zwei Leuchtstoffen versehen sind oder Hochdruck-Quecksilberdampf-Entladungslampen ohne Leuchtstoff siehe zum Beispiel US-4,048,537. Beide Lampenarten emittieren hauptsächlich im UV-A-Bereich. Dabei kann bei den Lampen mit Leuchtstoff vorteilhaft das UV-A-Spektrum durch die Zusammensetzung des Leuchtstoffes eingestellt werden, jedoch enthalten beide Lampen nachteiligerweise hochgiftiges Quecksilber, so dass sie aufwendig entsorgt werden müssen.

Die mit diesen Lampen erhaltenen Bräunungsresultate, insbesondere die Hautfarbe und die Dauer der Bräunung hängen vor allem von der spektralen Leistungsverteilung der UV-Quelle ab, da sowohl die Hautrötung (Erythema) als auch die sofortige und verzögerte Pigmentierung eine starke Abhängigkeit von der Wellenlänge des eingestrahlten UV-Lichts zeigen. Deshalb wird bei manchen Lampentypen ein zusätzlicher UV-B-Leuchtstoff hinzugeführt, um das Spektrum der Lampen in gewünschter Weise zu verändern. Das Spektrum der Lampen wird üblicherweise mit folgenden Verhältnissen ausgedrückt: UV-B / UV-A, UV-A₁ / UV-A₂ und Erythema-B / Erythema-A. Dabei bewegen sich die Größen üblicherweise in folgenden Wellenlängenbereichen: UV-A₁ = 340 bis 400 nm, UV-A₂ = 320 bis 340 nm, Erythema-B = 280 bis 320 nm, Erythema-A=320 bis 400 nm, UV-B = 280 bis 320 nm.

In jüngerer Zeit wurden Lampen entwickelt, deren Leuchtstoffzusammensetzung aus einer Mischung von LaPO₄:Ce (abgekürzt LAP genannt) und BaSi₂O₅:Pb (BSP) basiert. Diese Lampen zeigen ein erythemisches Effizienzspektrum, das dem der Sonne sehr ähnlich ist.

Obwohl bereits große Fortschritte im Bereich der Bräunungslampen gemacht wurden, weisen sie immer noch eine Reihe von Nachteilen auf, die direkt mit der Verwendung des Quecksilber-Plasmas verbunden sind. So liegen z.B. einige Spektrallinien des Quecksilber-Plasmas im UV-Bereich (297, 312,6 und 365 nm) und einige im sichtbaren Bereich (405, 435, 546 und 579 nm). Auf Grund der Anwesenheit dieser sichtbaren Quecksilberlinien erscheint das Licht dieser Lampen beim Betrieb bläulich, was vom Kunden nicht gewünscht wird. Die Quecksilberdampflinien im UV-Bereich stören zudem die freie Einstellbarkeit des UV-Spektrums mittels des Leuchtstoffs und müssen bei der Berechnung der Spezifikationsparameter einer solchen Lampe berücksichtigt werden.

Ein weiterer Nachteil ist die geringe Standfestigkeit dieser Lampen. So bildet sich z.B. durch Wechselwirkung des Quecksilbers mit bestimmten Leuchtstoffen, z.B. BSP, eine UV-Licht-absorbierende Schicht, die die UV-Lichtausbeute während der Lebensdauer der Lampe stark reduziert. Zudem ist das gewünschte UV-Licht auf Grund des Startverhaltens der Lampen nicht sofort verfügbar. Schließlich ist die Lampengeometrie beschränkt auf röhrenartige Formen, so dass ein Feld von nebeneinander angeordneten Lampen verwendet werden muss, um über eine größere Fläche, wie z.B. bei einer Sonnenbank, eine vernünftige Lichtverteilung zu bekommen. Selbst bei Anordnung mehrerer Lampen nebeneinander wird niemals eine perfekt homogene Lichtverteilung erzielt.

Davon ausgehend liegt der Erfindung die Aufgabe zugrunde, eine Lampe der eingangs genannten Art und ein Verfahren zum Herstellen einer solchen Lampe anzugeben, welche sich insbesondere für kosmetische und therapeutische Zwecke eignen, die es ermöglichen, die Vorteile einer Edelgas-Niederdruck-Entladungslampe zur Erzeugung von UV-Licht eines gewünschten Spektralbereichs zu nutzen.

Die Aufgabe wird gelöst durch die Verwendung einer Edelgas-Niederdruck-Entladungslampe mit einem mit Edelgas befüllten zumindest partiell UV-lichtdurchlässigen Entladungskörper, bei welcher der Entladungskörper zumindest partiell mit einem Leuchtstoffbeschichtet ist, der bei Anregung mit einem im Entladungskörper produzierten Anregungslicht UV-Licht abstrahlt, für kosmetische oder therapeutische Zwecke.

Ein großer Vorteil einer solche Lampe ist, dass nach der Zündung sofort Licht der gewünschten Art zur Verfügung stehen kann, während bei Quecksilberdampf-Endadungslampen zunächst das Quecksilber verdampft werden muss. Ein weiterer Vorteil der Lampe ist ihre mit üblicherweise mehr als 20.000 Betriebsstunden sehr hohe Lebensdauer. Zudem ist die Lampe umweltfreundlich, da sie kein giftiges Quecksilber enthält.

Da für Bräunungszwecke eine UV-Quelle mit einer Breitband-Emission im Bereich von 290 bis 400 nm ohne zusätzliche Plasma-Emissionslinien im UV- und/oder sichtbaren Bereich gewünscht ist, kann in der Entladungsröhre ein Edelgas wie z.B. Xenon oder Neon verwendet werden, das ausschließlich im VUV- und/oder UV-C-Bereich emittiert. Es kann auch sinnvoll sein, ein Gemisch dieser beiden Gase zu verwenden. Die erzeugten kurzen Wellenlängen können dann durch einen Leuchtstoff oder eine Mischung verschiedener Leuchtstoffe in geeignetes UV-A und/oder UV-B-Licht konvertiert werden. Dies hat den Vorteil, dass sich keine Plasmalinien im sichtbaren Bereich befinden, so dass das Licht der Lampe vom Benutzer nicht als unangenehm empfunden wird.

Bei einer bevorzugten Ausführungsform der Erfindung besteht der Entladungskörper zumindest partiell aus einem Glas, vorzugsweise aus einem Glas mit einer Durchlässigkeit von 20 bis 70 % für Licht der Wellenlänge 312,6 nm.

Die Erfindung ermöglicht vorteilhaft die Herstellung einer Lampe, bei der die spektrale Leistungsverreilung nur von dem verwendeten Leuchtstoff bzw. Leuchtstoffgemisch abhängt. Als besonders vorteilhaft hat es sich erwiesen, einen Leuchtstoff bzw. eine Kombination oder Mischung aus verschiedenen Leuchtstoffen zu verwenden, bei dem weniger als 1% des von ihm bzw. ihr bei Anregung mit einem im Entladungskörper produzierten Anregungslicht abgestrahlten Lichts Wellenlängen unter 290 nm, zwischen 1% und 10% des abgestrahlten Lichts Wellenlängen zwischen 290 und 320 nm bzw. weniger als 5% des abgestrahlten Lichts Wellenlängen über 400 nm besitzen.

Solche Abstrahlungseigenschaften erlauben beispielsweise Leuchtstoffe bzw. Kombination von Leuchtstoffen wie BaSi₂O₅:Pb (abgekürzt BSP genannt), CeMgAl₁₁O₁₉ (CAM), LaPO₄:Ce (LAP), SrB₄O₇:Eu (SBE) und (Sr, Ba)MgSi₂O₇:Pb (SMS). Diese Leuchtstoffe besitzen eine hohe Effizienz bei VUV-Anregung insbesondere im Bereich von 140 bis 190 nm, in dem die Edelgas-Entladungslampen hauptsächlich emittieren, und ihr Emissionsband liegt im UV-A und/oder UV-B-Bereich mit jeweils einer eher geringen Breite, so dass die Emission in einem Bereich konzentriert werden kann, in dem z.B. die Bräunungseffizienz besonders hoch ist. Durch die Anwendung einer Kombination eines UV-B-Leuchtstoffs, z.B. LAP, und eines UV-A-Leuchtstoffs, z.B. BSP, können die Spezifikationsparameter der Lampe so eingestellt werden, dass sich die gewünschte Annäherung an einen bestimmtes gewünschtes Spektrum ergibt.

Da im Regelfall das von der Lampe erzeugte Licht in eine Vorzugsrichtung abgestrahlt werden soll, kann auf Teilen des Entladungskörpers eine UV-Licht reflektierende Schicht, insbesondere eine MgO und/oder Al₂O₃ enthaltende Schicht aufgebracht sein, so dass das im Entladungskörper erzeugte hochenergetische UV-Licht auf bestimmte Abschnitte des Entladungskörpers, an denen es in niederenergetisches UV-Licht umgewandelt werden soll, gelenkt werden kann und die Lampe einen sehr hohen Wirkungsgrad und eine entsprechend hohe Bräunungseffizienz aufweist.

Die Lampe ermöglicht vorteilhaft ein insbesondere bei Bräunungslampen völlig neues, freies Design der Lampengeometrie, die z.B. gebogen oder flach sein kann, während die bekannten Quecksilberdampf-Entladungslampen immer röhrenartig gestaltet werden mussten. Damit wird es möglich, den Entladungskörper an die Umrisslinie einer mit der Lampe zu beleuchtenden Fläche anzupassen.

Darüber hinaus schlägt die Erfindung ein entsprechendes Herstellungsverfahren für eine derartige Lampe vor. Hierbei kann insbesondere eine Suspension des aufzubringenden Leuchtmittels präpariert und auf die Innenseite des Entladungskörpers aufgebracht werden. Hierfür eignet sich vorzugsweise ein Fließbeschichtungsverfahren, wobei als Beschichtungsgewicht sich die Spannweite zwischen 2 und 6 mg/cm² als vorteilhaft erwiesen hat. Anschließend wird die Suspension, beispielsweise durch Ausbrennen eines Bindemittels, fixiert, der Entladungskörper abgedichtet und befüllt.

Dementsprechend werden ein hierfür geeigneter Entladungskörper sowie ein Herstellungsverfahren für einen derartigen Entladungskörper beschrieben.

Eine derartige Entladungslampe kann insbesondere in einer UV-Licht-Bestrahlungsvorrichtung zur Anregung einer Hautpigmentbildung bzw. für kosmetische oder therapeutische Zwecke Verwendung finden. Die Erfindung schlägt die Verwendung einen Gasentladungslampe als Bräunungslampe vor, bei welcher ein Leuchtstoff von Licht mit einer Wellenlänge unter 200 nm angeregt wird. Die Verwendung der an sich für Bräunungszwecke ungeeigneten, in direktem Kontakt zur Haut sogar schädlichen Strahlung ermöglicht in überraschender Weise, das von dem Leuchtstoff abgestrahlte Licht wesentlich flexibler zu wählen und somit das abgestrahlte Spektrum geeignet und optimaler anzupassen.

Weitere Einzelheiten, Ziele und Vorteile der Erfindung ergeben sich aus der nachfolgenden rein beispielhaften und nicht-beschränkenden Beschreibung einiger Ausführungsbeispiele erfindungsgemäßer Lampen und Durchführungsformen erfindungsgemäßer Verfahren in Verbindung mit der Zeichnung. Es zeigen:
- Fig. 1: einen schematischen Querschnitt durch eine Ausführungsform einer erfindungsgemäßen röhrenartigen DBD(Dielectric Barrier Discharge)-Lampe für Bräunungszwecke mit außenliegenden, streifenartigen Elektroden,
- Fig. 2: einen schematischen Querschnitt durch eine flache DBD-Lampe für Bräunungszwecke mit äußeren streifenartigen Elektroden,
- Fig. 3: einen schematischen Querschnitt durch eine gebogene DBD-Lampe für Bräunungszwecke mit äußeren streifenartigen Elektroden,
- Fig. 4: das Emissionsspektrum einer Ein-Komponenten-DBD-Lampe mit BSP-Leuchtstoff in Standardglas,
- Fig. 5: das Emissionsspektrum einer Ein-Komponenten-DBD-Lampe mit SBE-Leuchtstoff in Standardglas,
- Fig. 6: das Emissionsspektrum einer Xenon-Gasentladungslampe mit einem SMS-Leuchtstoff und
- Fig. 7: das Emissionsspektrum einer Xenon-Gasentladungslampe mit einem Leuchtstoffgemisch aus 40 % LAP und 60 % BSP.

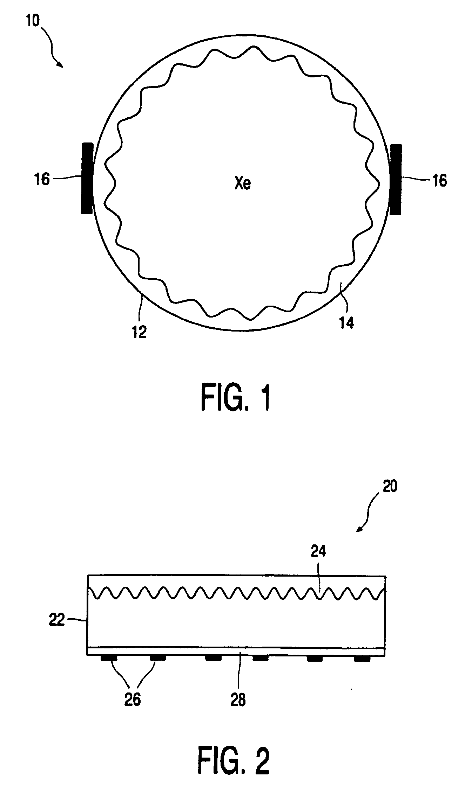

In der Fig. 1 ist eine in ihrer Gesamtheit mit 10 bezeichnete Edelgas-Niederdruck-Entladungslampe mit einer dielektrischen Sperrschicht gezeigt, wobei das Emissions-Maximum der Lampe im UV-A-Bereich liegt. Die Lampe weist einen gasdichten Entladungskörper 12 auf, der bei diesem Ausführungsbeispiel röhrenartig ausgebildet und mit Xenon befüllt ist. Der Entladungskörper 12 ist auf seiner Innenseite durchgängig mit einer lumineszenten Beschichtung 14 versehen, die wenigstens ein lumineszentes Material enthält, das im UV-A-Bereich (320 - 400 nm) emittiert. Ein zusätzliches lumineszentes Material, das im UV-B-Bereich (280 - 320 nm) emittiert, kann hinzugefügt werden, um das Verhältnis UV-B / UV-A zu justieren.

Auf der Außenseite des Entladungskörpers 12 sind zwei Elektroden 16, z.B. Al-Elektroden oder sog. ITO-Elektroden (ITO = Indium-Tin-Oxid = SnO₂:In), vorgesehen, wobei die ITO-Elektroden den Vorteil haben, transparent zu sein. Die solchermaßen aufgebaute, sogenannte DBD-(Dielectric-Barrier-Discharge)-Lampe besitzt eine hohe Leistungsdichte und eine lange Lebenszeit, die bei mehr als 20.000 Betriebsstunden liegen kann.

Das Plasmaemissionsspektrum dieser Lampe besteht nur aus einem engen Emissionsband, dessen Mitte bei Befüllung mit Xenon bei 172 nm liegt, wobei dann lediglich einige Linien im Infrarot-Bereich bei etwa 828 nm mit geringer Intensität emittiert werden. Das UV-Emissionsspektrum der Lampe hängt vorteilhaft nur von der Wahl der UV-A- und UV-B-Leuchtstoffe ab. Darüber hinaus bietet eine solche Lampe innerhalb weniger Millisekunden eine hundertprozentige Lichtleistung im VUV-Bereich (140 bis 190 nm).

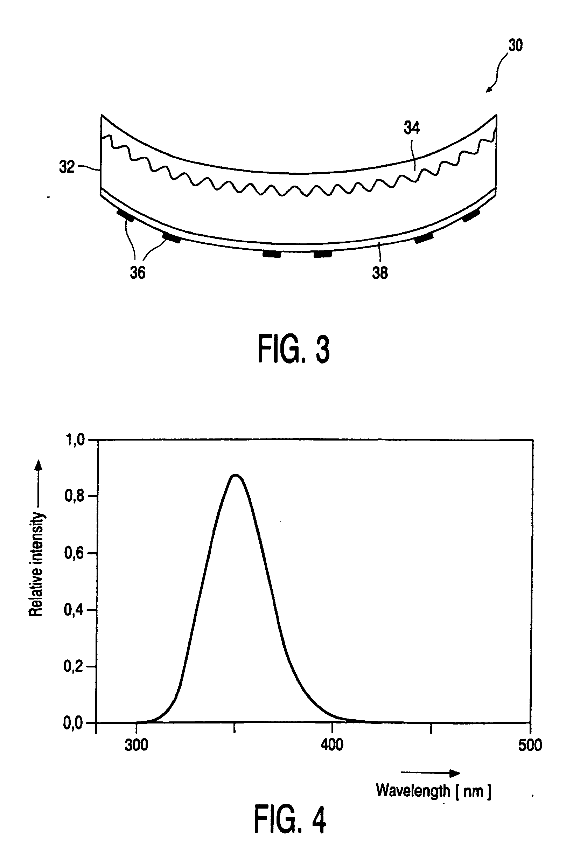

Da der Lampentyp nicht auf eine bestimmte, insbesondere röhrenartige Form beschränkt ist, ist es auch möglich Lampen mit einer flachen oder gebogenen Geometrie herzustellen, wie in den Fig. 2 und 3 gezeigt. Deshalb erlaubt es dieser Lampentyp, z.B. Sonnenbänke mit einer sehr homogenen Lichtverteilung herzustellen.

In den Fig. 2 und 3 ist jeweils ein schematischer Querschnitt durch eine flache DBD-Lampe 20 (Fig. 2) bzw. durch eine gebogene DBD-Lampe 30 (Fig. 3) gezeigt. Beide Lampen besitzen jeweils einen mit Edelgas befüllten Entladungskörper 22 bzw. 32, der auf seiner Innenseite partiell, nämlich im Bereich der gewünschten Abstrahlungsrichtung, mit einem Leuchtstoff oder einer Leuchtstoffkombination 24 bzw. 34 zur Umwandlung des im Betrieb der Lampe in dem Entladungskörper erzeugten hochenergetischen UV-Lichts in niederenergetisches UV-Licht beschichtet ist.

Auf der Außenseite der jeweiligen Entladungskörper sind streifenartige Elektroden 26 bzw. 36 vorgesehen.

Um den Wirkungsgrad der Lampen zu erhöhen, ist bei beiden Lampen auf der der gewünschten Abstrahlungsrichtung gegenüberliegenden Seite des Entladungskörpers ein UV-Reflektor 28 bzw. 38 vorgesehen. Ein solcher UV-Reflektor kann in unterschiedlicher, dem jeweiligen Einsatzzweck optimal angepasster Weise realisiert werden, z.B. durch Aufbringen einer Beschichtung auf den Entladungskörper oder in Form eines separaten UV-Reflektionsspiegels.

Im folgenden wird ein Beispiel zur Herstellung einer Ein-Komponenten-DBD-Lampe mit BSP-Leuchtstoff in Standardglas mit einer Transmission T_{312.6nm} von 35 % für Licht einer Wellenlänge von 312,6 nm angegeben:

Eine Suspension aus BSP wird auf einer Butylacetat-Basis mit Nitrozellulose als Bindemittel präpariert. Die Suspension wird in einem Fließbeschichtungsverfahren auf die Innenseite eines Entladungskörpers in Form einer Lampenröhre aus Standardglas mit 1 mm Dicke aufgebracht, was zu einer 35%-igen Transmission von Licht mit einer Wellenlänge von 312,6 nm bei einem typischen Gewicht der Leuchtstoff-Schicht von 2 bis 6 mg/cm² führt.

Das Bindemittel wird bei einem Heizzyklus mit Spitzentemperaturen zwischen 500 und 600 Grad ausgebrannt. Die Glasröhre wird abgedichtet und mit Xenon befüllt. Der Gasdruck des Xenons sollte zwischen 200 und 300 mbar liegen. Al-Elektroden werden an der Außenseite der Lampen durch Adhäsion oder Tauchen aufgebracht.

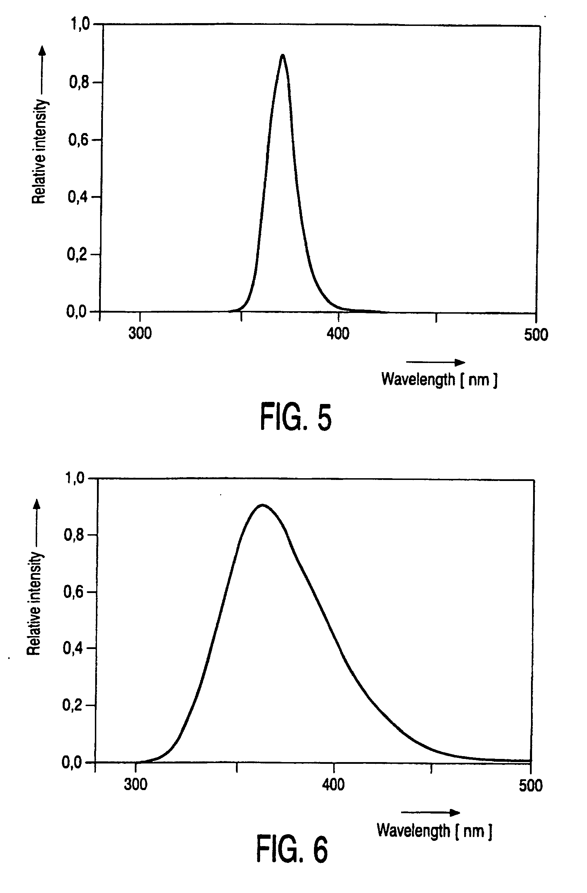

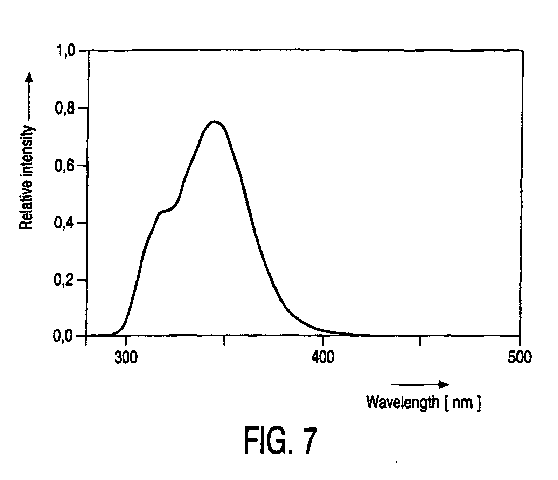

Eine solche Lampe kann dann mit 6 kV und 25 kHz bei rechteckiger Wechselspannung betrieben werden. Das Emissionsspektrum einer solchen Lampe zeigt Fig. 4, während in den Fig. 5, 6 und 7 die Emissionsspektren einer Ein-Komponenten-DBD-Lampe mit SBE-Leuchtstoff in Standardglas (Fig. 5), einer Xenon-Gasentladungslampe mit einem SMS-Leuchtstoff (Fig. 6) und einer Xenon-Gasentladungslampe gezeigt ist, auf deren Entladungskörper eine Mischung aus 40 % LAP und 60 % BSP-Leuchtstoff aufgebracht wurde (Fig. 7). Die Verfahren zur Herstellung dieser Lampen entsprechen dabei jeweils dem beschriebenen Verfahren.

## Patentansprüche

1. Verwendung einer zur Erzeugung ultravioletten Lichts vorgesehenen quecksilberfreien Edelgas-Niederdruck-Entladungslampe mit einem mit Edelgas befüllten zumindest partiell UV-lichtdurchlässigen Entladungskörper, der zumindest partiell mit einem Leuchtstoff beschichtet ist, der bei Anregung mit einem im Entladungskörper produzierten Anregungslicht UV Licht abstrahlt, für kosmetische oder therapeutische Zwecke.

2. Verwendung nach Anspruch 1,
**dadurch gekennzeichnet,**
**dass** der Leuchtstoff derart ausgebildet ist, dass zwischen 1% und 10% der Leistung des von ihm bei Anregung mit einem im Entladungskörper produzierten Anregungslicht abgestrahlten Lichts im Wellenlängenbereich zwischen 290 und 320 nm liegt.

3. Verwendung nach Anspruch 1 oder 2,
**dadurch gekennzeichnet,**
**dass** auf Teilen des Entladungskörpers eine UV-Licht reflektierende Schicht, insbesondere eine MgO und/oder Al₂O₃ enthaltende Schicht, aufgebracht ist.

4. Verwendung nach einem der Ansprüche 1 bis 3,
**dadurch gekennzeichnet,**
**dass** das im Entladungskörper produzierte Anregungslicht Wellenlängen im VUV-Bereich besitzt.

5. Verwendung nach einem der Ansprüche 1 bis 4,
**dadurch gekennzeichnet,**
**dass** der Entladungskörper mit Xenon oder Neon befüllt ist.

6. Verwendung nach einem der Ansprüche 1 bis 5,
**dadurch gekennzeichnet,**
**dass** der Entladungskörper zumindest partiell aus einem Glas, vorzugsweise aus einem Glas mit einer Durchlässigkeit von 20 bis 70 % für Licht der Wellenlänge 312,6 nm besteht.

7. Verwendung nach einem der Ansprüche 1 bis 6,
**dadurch gekennzeichnet,**
**dass** der Leuchtstoff derart ausgebildet ist, dass weniger als 1% der Leistung des von ihm bei Anregung mit einem im Entladungskörper produzierten Anregungslicht abgestrahlten Lichts im Wellenlängenbereich unter 290 nm liegt.

8. Verwendung nach einem der Ansprüche 1 bis 7,
**dadurch gekennzeichnet,**
**dass** der Leuchtstoff derart ausgebildet ist, dass weniger als 5% der Leistung des von ihm bei Anregung mit einem im Entladungskörper produzierten Anregungslicht abgestrahlten Lichts im Wellenlängenbereich über 400 nm liegt.

9. Verwendung nach einem der Ansprüche 1 bis 8,
**dadurch gekennzeichnet,**
**dass** der Leuchtstoff zumindest einen Leuchtstoff, vorzugsweise eine Kombination von Leuchtstoffen, aus der folgenden Gruppe von Leuchtstoffen enthält: BaSi₂O₅:Pb (BSP), CeMgAl₁₁O₁₉ (CAM), LaPO₄:Ce (LAP), SrB₄O₇:Eu (SBE), (Sr, Ba)MgSi₂O₇:Pb (SMS).

10. Verwendung nach einem der Ansprüche 1 bis 9,
**dadurch gekennzeichnet,**
**dass** der Entladungskörper nicht röhrenförmig ist.

11. Verwendung nach einem der Ansprüche 1 bis 10,
**dadurch gekennzeichnet,**
**dass** zwei der drei Dimensionen des Entladungskörpers, insbesondere seine Länge und Breite, wesentlich größer sind als seine dritte Dimension, insbesondere seine Dicke.

12. Verwendung nach einem der Ansprüche 1 bis 11,
**dadurch gekennzeichnet,**
**dass** der Entladungskörper an die Umrisslinie einer mit der Lampe zu beleuchtenden Fläche angepasst ist.

## Claims

1. The use of a mercury-free noble-gas low-pressure discharge lamp for generating ultraviolet light for cosmetic or therapeutic purposes, which lamp has a discharge vessel that is filled with a noble gas and that is at least partly transparent to UV light, wherein the discharge vessel is at least partly coated with a phosphor which radiates UV light when excited by excitation light produced in the discharge vessel.

2. The use as claimed in claim 1, **characterized in that** the phosphor is formed such that between 1% and 10% of the light power radiated thereby upon excitation by an excitation light produced in the discharge vessel has wavelengths between 290 and 320 nm.

3. The use as claimed in claim 1 or 2, **characterized in that** a UV light reflecting layer, in particular a layer comprising MgO and/or Al₂O₃, is provided on portions of the discharge vessel.

4. The use as claimed in any one of the claims 1 to 3, **characterized in that** the excitation light produced in the discharge vessel has wavelengths in the VUV range.

5. The use as claimed in any one of the claims 1 to 4, **characterized in that** the discharge vessel is filled with xenon or neon.

6. The use as claimed in any one of the claims 1 to 5, **characterized in that** the discharge vessel is at least partly made of a glass, preferably of a glass having a transmittance of 20 to 70% for light of 312.6 nm wavelength.

7. The use as claimed in any one of the claims 1 to 6, **characterized in that** the phosphor is formed such that less than 1% of the light power radiated thereby upon excitation by an excitation light produced in the discharge vessel has wavelengths below 290 nm.

8. The use as claimed in any one of the claims 1 to 7, **characterized in that** the phosphor is formed such that less than 5% of the light power radiated thereby upon excitation by an excitation light produced in the discharge vessel has wavelengths above 400 nm.

9. The use as claimed in any one of the claims I to 8, **characterized in that** the phosphor comprises at least one luminescent material, preferably a combination of luminescent materials, chosen from the following group of luminescent materials: BaSi₂O₅:Pb (BSP), CeMgAl₁₁O₁₉ (CAM), LaPO₄:Ce (LAP), SrB₄O₇:Eu (SBE), (Sr,Ba)MgSi₂O₇:Pb (SMS).

10. The use as claimed in any one of the claims 1 to 9, **characterized in that** the discharge vessel is not tubular in shape.

11. The use as claimed in any one of the claims 1 to 10, **characterized in that** two of the three dimensions of the discharge vessel, in particular its length and width, are substantially greater than its third dimension, in particular its thickness.

12. The use as claimed in any one of the claims 1 to 11, **characterized in that** the discharge vessel is adapted to the contours of a surface to be irradiated with the lamp.

## Revendications

1. Utilisation cosmétique ou thérapeutique d'une lampe à décharge basse pression à gaz rare sans mercure prévue pour la production d'une lumière ultraviolette avec un corps de décharge perméable à la lumière U.V., du moins partiellement, et rempli de gaz rare, qui est revêtu, du moins partiellement, d'une substance luminescente qui diffuse de la lumière U.V. en cas d'excitation avec une lumière d'excitation produite dans le corps de décharge.

2. Utilisation selon la revendication 1,
**caractérisée en ce**
**que** la substance luminescente est conçue de telle sorte qu'entre 1 % et 10 % de la puissance de la lumière diffusée par elle en cas d'excitation par une lumière d'excitation produite dans le corps de décharge se situe dans la gamme de longueur d'ondes située entre 290 et 320 nm.

3. Utilisation selon l'une des revendications 1 ou 2,
**caractérisée en ce**
**qu'**une couche réfléchissant la lumière U.V., en particulier une couche contenant du MgO et/ou de l'Al₂O₃ est appliquée sur des parties du corps de décharge.

4. Utilisation selon l'une des revendications 1 à 3,
**caractérisée en ce**
**que** la lumière d'excitation produite dans le corps de décharge possède des longueurs d'onde dans la gamme VU.V..

5. Utilisation selon l'une des revendications 1 à 4,
**caractérisée en ce**
**que** le corps de décharge est rempli de xénon ou de néon.

6. Utilisation selon l'une des revendications 1 à 5,
**caractérisée en ce**
**que** le corps de décharge se compose, du moins partiellement, d'un verre, de préférence d'un verre ayant une perméabilité de 20 à 70 % pour la lumière de longueur d'onde de 312,6 nm.

7. Utilisation selon l'une des revendications 1 à 6,
**caractérisée en ce**
**que** la substance luminescente est conçue de telle sorte que moins de 1 % de la puissance de la lumière diffusée par elle en cas d'excitation avec une lumière d'excitation produite dans le corps de décharge se situe dans une gamme de longueurs d'onde inférieure à 290 nm.

8. Utilisation selon l'une des revendications 1 à 7,
**caractérisée en ce**
**que** la substance luminescente est conçue de telle sorte que moins de 5 % de la puissance de la lumière diffusée par elle en cas d'excitation avec une lumière d'excitation produite dans le corps de décharge se situe dans une gamme de longueurs d'onde supérieure à 400 nm.

9. Utilisation selon l'une des revendications 1 à 8,
**caractérisée en ce**
**que** la substance luminescente contient au moins une substance luminescente, de préférence une combinaison de substances luminescentes à partir du groupe suivant de substances luminescentes : BaSi₂O₅:Pb (BSP), CeMgAl₁₁O₁₉ (CAM), LaPO₄:Ce (LAP), SrB₄O₇:Eu (SBE), (Sr, Ba)MgSi₂O₇:Pb (SMS).

10. Utilisation selon l'une des revendications 1 à 8,
**caractérisée en ce**
**que** le corps de décharge n'est pas tubulaire.

11. Utilisation selon l'une des revendications 1 à 10,
**caractérisée en ce**
**que** deux des trois dimensions du corps de décharge, en particulier sa longueur et sa largeur, sont essentiellement supérieures à sa troisième dimension, en particulier son épaisseur.

12. Utilisation selon l'une des revendications 1 à 11,
**caractérisée en ce**
**que** le corps de décharge est adapté à la ligne de contour d'une surface à exposer avec la lampe.
